# EUROPEAN PATENT SPECIFICATION

(11) **EP 3 044 312 B1**
(45) Date of publication and mention of the grant of the patent: **21.03.2018**
(21) Application number: 14761658.5
(22) Date of filing: 10.09.2014
(51) Int. Cl.: C12N 9/02

(54) **REAGENTS AND METHODS FOR THE EXPRESSION OF OXYGEN-SENSITIVE PROTEINS**
REAGENZIEN UND VERFAHREN ZUR EXPRESSION VON SAUERSTOFFEMPFINDLICHEN PROTEINEN
RÉACTIFS ET PROCÉDÉS POUR L'EXPRESSION DE PROTÉINES SENSIBLES À L'OXYGÈNE

(30) Priority: 11.09.2013 EP 13382352
(43) Date of publication of application: 20.07.2016
(73) Proprietor: Universidad Politécnica De Madrid, 28040 Madrid (ES)
(72) Inventor: RUBIO HERRERO, Luis Manuel, E-28223 Pozuelo de Alarcón (ES); LÓPEZ TORREJÓN, Gema, E-28223 Pozuelo de Alarcón (ES); JIMÉNEZ VICENTE, Emilio, E-28223 Pozuelo de Alarcón (ES); BUESA GALIANO, Jose María, E-28223 Pozuelo de Alarcón (ES)
(74) Representative: ABG Patentes, S.L.
(86) International application number: PCT/EP2014/069261
(87) International publication number: WO 2015/036419

(56) References cited:
- BERMAN ET AL: "Expression of nitrogen fixation genes in foreign hosts. Assembly of nitrogenase Fe protein in Escherichia coli and in yeast.", JOURNAL OF BIOLOGICAL CHEMISTRY, vol. 260, no. 9, 1 May 1985 (1985-05-01), pages 5240-5243, XP055100305, ISSN: 0021-9258
- A. ZAMIR ET AL: "Stable chromosomal integration of the entire nitrogen fixation gene cluster from Klebsiella pneumoniae in yeast.", PROCEEDINGS OF THE NATIONAL ACADEMY OF SCIENCES, vol. 78, no. 6, 1 June 1981 (1981-06-01), pages 3496-3500, XP055100183, ISSN: 0027-8424, DOI: 10.1073/pnas.78.6.3496
- NINA PETROVA ET AL: "NifH and NifM Proteins Interact as Demonstrated by the Yeast Two-Hybrid System", BIOCHEMICAL AND BIOPHYSICAL RESEARCH COMMUNICATIONS, vol. 270, no. 3, 1 April 2000 (2000-04-01) , pages 863-867, XP055100148, ISSN: 0006-291X, DOI: 10.1006/bbrc.2000.2499
- VAN STEEG H ET AL: "TARGETING EFFICIENCY OF A MITOCHONDRIAL PRE-SEQUENCE IS DEPENDENT ON THE PASSENGER PROTEIN", EMBO (EUROPEAN MOLECULAR BIOLOGY ORGANIZATION) JOURNAL, vol. 5, no. 13, 1986, pages 3643-3650, XP055100444, ISSN: 0261-4189

### SEQUENCE LISTING

<110> Universidad Politécnica de Madrid
<120> REAGENTS AND METHODS FOR THE EXPRESSION OF OXYGEN-SENSITIVE PROTEINS
<130> P9452PC00
<150> EP13382352
   <151> 2013-09-11
<160> 7
<170> PatentIn version 3.5
<210> 1
   <211> 20
   <212> PRT
   <213> Artificial Sequence
<220>
   <223> Mitocondrial targeting peptide of S. cerevisiae superoxide dismutase (SOD)
<400> 1
   Met Phe Ala Lys Thr Ala Ala Ala Asn Leu Thr Lys Lys Gly Gly Leu
   1               5                   10                  15
   Ser Leu Leu Ser
               20
<210> 2
   <211> 40
   <212> PRT
   <213> Artificial Sequence
<220>
   <223> mitocondrial targeting peptide comprising two tanden copies of S. cerevisiae SOD
<400> 2
   Met Phe Ala Lys Thr Ala Ala Ala Asn Leu Thr Lys Lys Gly Gly Leu
   1               5                   10                  15
   Ser Leu Leu Ser Met Phe Ala Lys Thr Ala Ala Ala Asn Leu Thr Lys
               20                  25                  30
   Lys Gly Gly Leu Ser Leu Leu Ser
           35                  40
<210> 3
   <211> 6
   <212> PRT
   <213> Artificial Sequence
<220>
   <223> tag sequence
<400> 3
   Ala His Gly His Arg Pro
   1               5
<210> 4
   <211> 14
   <212> PRT
   <213> Artificial Sequence
<220>
   <223> tag sequence
<400> 4
   Pro Ile His Asp His Asp His Pro His Leu Val Ile His Ser
   1               5                   10
<210> 5
   <211> 7
   <212> PRT
   <213> Artificial Sequence
<220>
   <223> tag sequence
<220>
   <221> UNSURE
   <222> (5)..(6)
   <223> Xaa can be any naturally occurring amino acid
<400> 5
   Gly Met Thr Cys Xaa Xaa Cys
   1               5
<210> 6
   <211> 6
   <212> PRT
   <213> Artificial Sequence
<220>
   <223> tetracysteine motif
<220>
   <221> UNSURE
   <222> (3)..(4)
   <223> Xaa can be any naturally occuring amino acid
<400> 6
   Cys Cys Xaa Xaa Cys Cys
   1               5
<210> 7
   <211> 1749
   <212> DNA
   <213> Artificial sequence
<220>
   <223> Polynucleotide encoding the fusion protein sod2- GFP-NifH
<400> 7
   atgttcgcta agacagccgc cgctaacttg acaaagaagg gtggtttgtc attgctctct 60
   atgttcgcaa agactgccgc agctaactta actaagaaag gtggtttgtc attgctcagt 120
   atgtcaaagg gtgaagaatt gtttactggt gttgtaccaa tcttggtaga attagatggt 180
   gacgtcaatg gtcataaatt ctccgttagt ggtgaaggtg aaggtgacgc aacatacggt 240
   aaattgacct tgaagtttat atgtactact ggtaaattgc cagttccttg gccaacattg 300
   gtaaccactt ttggttatgg tgttcaatgc ttcgccagat accctgatca tatgaaacaa 360
   cacgactttt tcaagtccgc tatgccagaa ggttacgttc aagaaagaac tatttttctt 420
   aaggatgacg gtaactacaa gaccagagct gaagtaaagt tcgaaggtga cactttggtc 480
   aacagaatcg aattgaaggg tatcgatttc aaggaagacg gtaacatatt gggtcataag 540
   ttggaataca attacaactc acacaacgta tacatcatgg ctgataagca aaagaatggt 600
   attaaagtca acttcaagat cagacataac atcgaagatg gttccgttca attagcagac 660
   cactatcaac aaaatacccc tattggtgac ggtcctgttt tgttaccaga caaccattac 720
   ttatccactc aaagtgcttt gtctaaagat ccaaatgaaa agagagacca tatggtcttg 780
   ttagaatttg ttactgctgc aggtattaca cacggtatgg atgaattgta taaagccgcc 840
   gcggggacaa tggccatgag acaatgtgct atctacggta aaggtggtat tggtaaaagt 900
   acaaccactc aaaacttggt tgccgcttta gcagaaatgg gtaaaaaggt catgattgtt 960
   ggttgcgatc ctaaggccga ctcaactaga ttgatattac actccaaagc tcaaaataca 1020
   atcatggaaa tggcagccga agcaggtacc gttgaagatt tggaattgga agacgtattg 1080
   aaggccggtt atggtggtgt aaaatgtgtc gaatctggtg gtcctgaacc aggtgtcggt 1140
   tgcgcaggta gaggtgttat aactgccatc aatttcttgg aagaagaagg tgcatacgaa 1200
   gatgacttag atttcgtttt ctacgatgta ttgggtgacg tcgtttgtgg tggttttgct 1260
   atgccaatta gagaaaacaa ggcacaagaa atctatatag tttgcagtgg tgaaatgatg 1320
   gctatgtacg ctgcaaacaa catctctaag ggtatagtca agtacgccaa ttctggttca 1380
   gttagattgg gtggtttgat ctgtaactca agaaacactg atagagaaga cgaattgatt 1440
   atagccttag ctaacaaatt gggtacacaa atgatccatt tcgtacctag agataatgta 1500
   gtccaaagag ctgaaatcag aagaatgaca gttatcgaat atgatccaaa agctaagcaa 1560
   gcagacgaat acagagcatt ggccagaaag gttgtagata ataagttgtt agttatacct 1620
   aacccaatca ctatggatga attggaagaa ttgttgatgg aattcggtat catggaagtt 1680
   gaagatgaat ctatagtcgg taaaactgct gaagaagtag gtagtagtgg ttgttgccca 1740
   ggttgttgt 1749

## Description

### FIELD OF THE INVENTION

The invention relates to the field of genetics and, more particularly, to methods for expressing an oxygen-sensitive protein in a eukaryotic cell and for *in vitro* reconstitution of active nitrogenase complex.

### BACKGROUND OF THE INVENTION

Nitrogen is essential in plant development and a limiting factor in plant growth. It represents about 2% of the total plant dry matter that enters the food chains. Nevertheless, plants cannot directly access nitrogen gas (N₂) which makes up 78% of the atmosphere. In order for nitrogen gas to be used for growth it must first be fixed (i.e. reduced by hydrogen to ammonia) and be available in the combined form of ammonium (NH₄⁺) or nitrate (NO₃⁻). Plants adsorb said combined forms of nitrogen through their roots.

It is known that nitrogen is the main limiting factor of agricultural plant cultivation since the supply of nitrogen by fertilization is albeit efficient but expensive and is accompanied by an extreme environmental pollution due to the inefficient use of nitrogen by plants. Moreover, manufacturing nitrogen fertilizers requires six times more energy than needed to produce either phosphor or potassium fertilizers. The worldwide spreading conception of cultivation, the conception of sustainable development gives preference to the production based on internal resources instead of using external ones.

The so-called aerobic nitrogen-fixing bacteria, the members of genera *Azomonas*, *Azotobacter*, *Beijerinckia* and *Derxia* belonging to the family of Azotobactereceae (Bergey's Manual of determinative Bacterology, 8th ed., 1974), are capable of an efficient nitrogen-fixation even at atmospheric oxygen levels by the action of an evolutionarily conserved enzyme complex called nitrogenase. This complex is composed of two enzymes: a dinitrogenase and a dinitrogenase reductase (Bulen et al, Proc. Natl. Acad. Sci. U.S.A., 1966 56: 979-986). However, they are unable in the nature to be incorporated into the inner tissue spaces of plants (Azotobactereceae: The taxonomy and Ecologist of the Aerobic Nitrogen Fixing Bacteria, Academic Press, New York, 1979) and to spread in the intercellular spaces although it could be proven that, when settling down on the roots or on the outer surfaces of leaves, these species are capable to provide the nitrogen demand of some plants to a significant or whole extent (J. Gen. Microbiol. 71, 103-116, 1972; J. Gen. Appl. Microbiol. 25, 261-271, 1979; Can. J. Bot. 69, 2296-2298, 1991). Both protein components of nitrogenase are extremely sensitive to oxygen and the bacteria fixing nitrogen aerobically have evolved a variety of strategies to protect nitrogenase from oxygen poisoning. Among the members of the genus *Azotobacter,* there are three mechanisms for nitrogenase protection. These are conformational protection, respiratory regulation and oxygen permeability (Kennedy and Toukdarian, Annu. Rev. Microbiol., 1987).

The so-called microaerophilic diazotrophs, fixing the nitrogen at low oxygen levels, are only able to form intercellular endosymbioses (Nitrogen-fixing Bacteria in Nonleguminous Crop Plants, Sci. Tech. Publishers/Springer-Verlag, Madison, WI, pp. 84-88, 1987) which, however, can fix nitrogen gas only in the roots, i.e. far from the site of photosynthesis. The best known example of beneficial plant-bacteria association for nitrogen nutrition occurs in nodulating plants (Sprent and James, Plant Physiology, 2007, 144:575-581).

Berman et al., 1985 J. of Biol. Chem., 260(9): 5240-5243 describes that *Escherichia coli* or the yeast *Saccharomyces cerevisiae,* transformed with the *K. pneumoniae* nifH gene in suitable expression vectors, synthesize the oxygen-sensitive NifH polypeptide.

Non-nodulating plants and nitrogen fixing bacteria can establish functional associations. *Azoarcus sp.* BH72 fixes nitrogen under microaerobic conditions. At nanomolar oxygen concentrations, these bacterial cells can shift into a state of higher activity of nitrogen fixation and respiratory efficiency in which intracytoplasmic membrane stacks (diazosomes) related to nitrogen fixation are formed, and the iron protein of the nitrogenase is highly enriched (Hurek et al., J. of Bacteriology, 1994, 176: 1913-1923; Hurek et al., J. of Bacteriology, 1994, 176:4726-4733;). Transcriptional fusion of the nitrogenase *nifH* gene promoter to green fluorescent protein reported high levels of nitrogenase gene expression in *Azoarcus sp.* BH72 within rice roots (Egener et al., Mol. Plant Microbe Interact, 1998, 11:71-75). In addition, molecular ecological methods were developed to assess *nifH* mRNA expression within Kallar grass (*Leptochloa fusca, Poaceae*) plants inoculated with this bacterium. Screening for the *nifH* gene by *nifH*-specific reverse transcription-PCR in root mRNA, showed that *Azoarcus sp.* BH72 expresses nitrogenase genes inside the plant root system (Hurek et al., Mol. Plant Microbe Interact, 2002, 15:233-242).

Genetic transformation of plants with genes of interest is a common technique used in order to make plants resistant to pest and agents which causing harm to cultures, to producing certain nutrients or pharmaceutical agents such vaccines and to improve the growth of these plants to assist in farmer efficiency. However, a genetically engineered plant capable of fixing nitrogen has not been produced yet. Two main barriers have impaired this approach: the known sensitivity of nitrogenase to oxygen, which is the byproduct of plant photosynthesis and the genetical and biochemical complexity of nitrogenase biosynthesis.

Patent application US 3704546 provides an aseptic procedure for symbiotic fixation of gaseous nitrogen in which the first step is the aseptic growth of undifferentiated plant cells derived from members of the Leguminosae in a suitable medium. This method is based on the contacting of undifferentiated plant cells contained in a liquid medium having the necessary nutrient materials present and bacteria of the genus *Rhizobium*. The plant cells and bacteria in the liquid culture medium are then incubated together for a suitable time and at a suitable temperature to allow microorganisms to enter and occupy the plant cells. Further growth and development of the plant cells with their contained microorganisms is accomplished in a medium having substantially no growth stimulating activity on the plant cells. The greatest nitrogen-fixing activity occurs in this latter stage.

Two studies have been carried out to co-cultivate *Azotobacter* species with plant cells under *in vitro* conditions. In the first experiment (Carlson et al., Nature 252, 393-395; 1974) an adenine-auxotrophic *Azotobacter vinelandii* mutant strain was co-cultivated with carrot cells on a sucrose-containing medium, which was nitrogen-free or contained nitrogen in an amount insufficient for plant growth. The thus-obtained mixed callus cultures kept growing for 18 months, showed nitrogen-fixing activity and the bacteria could be observed among the living plant cells. This method has, however, several drawbacks: an auxotrophic mutant strain of *Azotobacter* species is used which is difficult to isolate due to its high number of "chromosomes" (Sadoff et al., J. Bacteriol., 138, 871-877, 1979); the growth of the cultures is slow; the system is unstable in the presence of nitrogen; no plant could be regenerated; the applicability of the method is limited to a few intensely studied species because of the low number of auxotrophic strains.

In the other experiment (Vasil et al., Z. Pflanzenphysiol. Bd. 95, 141-147, 1979) the bacteria engulfed the plant tissue and destroyed it. Thus, it became impossible to cultivate for a long period and to develop a nitrogen-fixing plant.

Thus, it would be advantageous provide a method which allow plants fix their own nitrogen avoiding interactions of plants with specific symbiotic or associative nitrogen fixing bacteria.

### BRIEF SUMMARY OF THE INVENTION

In a first aspect, the invention relates to a polynucleotide encoding a fusion protein which comprises an oxygen-sensitive protein and a mitochondrial targeting peptide, wherein said oxygen-sensitive protein is NifH..

In another aspect, the invention relates to a polynucleotide sequence comprising a nucleotide sequence which encodes the fusion protein of the invention operatively linked to suitable transcriptional or translational regulatory elements.

In another aspect, the invention refers to an expression vector comprising the polynucleotide of the invention.

In another aspect, the invention refers to a eukaryotic cell comprising the polynucleotide of the invention or the expression vector of the invention.

In another aspect, the invention relates to a method to express NifH in a eukaryotic cell comprising the steps of:
i) introducing into said cell a polynucleotide according to the invention or a vector comprising said polynucleotide,
ii) growing said cell under conditions allowing the expression of said oxygen-sensitive protein and, if desired;
iii) purifying said oxygen-sensitive protein under anaerobic conditions.

In another aspect, the invention relates to a method for *in vitro* reconstitution of active nitrogenase protein complex comprising the steps of:
i) introducing into said cell a polynucleotide according to the invention or a vector according to the invention wherein the oxygen-sensitive protein is NifH and wherein the cell further comprises a nucleotide sequence which encodes a fusion protein comprising the NifM protein and a mitochondrial targeting peptide,
ii) growing said cell under conditions allowing the expression of NifH and NifM proteins,
iii) purifying NifH protein from said cells under anaerobic conditions and
iv) contacting said purified NifH of step (iii) with the NifDK complex.

In another aspect, the invention refers to a polynucleotide of the invention, an expression vector of the invention or a eukaryotic cell of the invention for use for expressing an oxygen-sensitive protein.

In another aspect, the invention refers to a kit comprising
i) a polynucleotide of the invention or an expression vector of the invention; and
ii) reagents to carry out the method to express an oxygen-sensitive protein in a eukaryotic cell and/or the method for *in vitro* reconstitution of active nitrogenase protein complex.

### BRIEF DESCRIPTION OF THE DRAWINGS

**Figure 1****.** Fluorescence quantification of NifH-yEGFP and NifM-mKO fusion proteins expressed in *Saccharomyces cerevisiae.*
**Figure 2****.** Aconitase activity determination in cell free extract of NifH and NifM induced proteins in *Saccharomyces cerevisiae*.
**Figure 3****.** *In vitro* nitrogenase activity estimated by the acetyle reduction assay. (A) NifH purified from recombinant yeast (concentrated sample) (B) NifH purified from recombinant yeast (diluted sample), (1 to 4) NifDK activity titration with NifH. A: 0,8 mg/ml yNifH; B: 0,08 mg/ml yNifH; 1: 0 mg/ml (in the absence of NifH); 2: 16 mg/ml (NifH/NifDK ratio of 1:16); 3: 40 mg/ml (NifH/NifDK ratio of 1:41); 4: 80 mg/ml (NifH/NifDK ratio of 1:82). Both components were purified from *Azotobacter vinelandii.*

### DETAILED DESCRIPTION OF THE INVENTION

The authors of the present invention have developed an efficient protein expression system that allows the expression of oxygen sensitive proteins in eukaryotic cells under aerobic conditions. This expression system is based on the expression of the oxygen sensitive protein in the yeast mitochondria. The authors of the present invention have observed that, surprisingly, it is possible to express oxygen sensitive proteins in the mitochondria and recover the protein in an active form. Thus, as shown in the examples of the present invention, the NifH component of the nitrogenase has been purified from the mitochondria of yeast cells expressing a construct that directs NifH into the mitochondria. The NifH is found in active form, as it is capable of catalysing NH3 formation in the presence of the second member of the nitrogenase complex (the NifDK complex).

### Polynucleotide of the invention

In a first aspect, the invention relates to a polynucleotide encoding a fusion protein which comprises an oxygen-sensitive protein and a mitochondrial targeting peptide, wherein said oxygen-sensitive protein is NifH.

The term "polynucleotide" as used herein relates to a polymer formed by a variable number of monomers wherein the monomers are nucleotides, including ribonucleotides as well as deoxyribonucleotides. The polynucleotides include monomers modified by methylation as well as unmodified forms. The terms "polynucleotide" and "nucleic acid" are used indiscriminately in the present invention and include mRNA, cDNA and recombinant polynucleotides. As used in the present invention, polynucleotides are not limited to polynucleotides as they appear in nature, and also include polynucleotides where unnatural nucleotide analogues and inter-nucleotide bonds appear. Non-limitative examples of this type of unnatural structures include polynucleotides wherein the sugar is different from ribose, polynucleotides wherein the phosphodiester bonds 3'-5' and 2'-5' appear, polynucleotides wherein inverted bonds (3'-3' and 5'-5') appear and branched structures. Also, the polynucleotides of the invention include unnatural inter-nucleotide bonds such as peptide nucleic acids (PNA), locked nucleic acids (LNA), C1-C4 alkylphosphonate bonds of the methylphosphonate, phosphoramidate, C1-C6 alkylphosphotriester, phosphorothioate and phosphorodithioate type. In any case, the polynucleotides of the invention maintain the capacity to hybridize with target nucleic acids in a similar way to natural polynucleotides.

The term "fusion protein" as used herein, relates to proteins generated by gene technology which consist of two or more functional domains derived from different proteins. A fusion protein may be obtained by conventional means (e.g. by means of gene expression of the nucleotide sequence encoding for said fusion protein in a suitable cell). The fusion protein of the invention comprises an oxygen-sensitive protein and a mitochondrial targeting peptide.

The term "oxygen-sensitive protein" as used herein, relates to which are readily oxidized and rendered inactive when exposed to oxic conditions. In a preferred embodiment, the oxygen-sensitive protein is an anaerobic enzyme. Many of such proteins contain metal complexes that are sensitive to the presence of oxygen. Without limitation, such proteins include proteins required for the molybdenum nitrogenases (e.g. nifH, nifDK, nifQ, nifB, nifEN, nifU); vanadium nitrogenases (e.g. vnfH, vnfDGK, vnfEN, nfuV), iron nitrogenases (e.g. anfH, anfDGK, nfuA), carbon monoxide dehydrogenase (e.g. cooS, cooF,), iron hydrogenase (e.g. hydA, hydE, hydG), nickel-iron hydrogenase (e.g. hycE), acetogenesis enzymes (e.g. pyruvate ferredoxin oxidoreductase (PFOR), acetyl-ScoA synthase), SAM radical enzymes from bacterial origin (e.g. MiaB, Biotin synthase, RimO), vinyl chloride reductase; 1,2-dichloroethene reductive dehalogenase; trichloroethene reductive dehalogenase; tetrachloroethene reductive dehalogenase; benzylsuccinate synthase; ethylbenzene dehydrogenase; oxygen-sensitive ribonucleoside triphosphate reductase, and the like.

Also disclosed are oxygen-sensitive proteins from a prokaryotic organism, preferably from bacteria. Said bacteria may belong to nitrogen-fixing bacteria, including free living nitrogen fixing bacteria, associative nitrogen fixing bacteria and symbiotic nitrogen fixing bacteria. Free living nitrogen fixing bacteria are capable of fixing significant levels of nitrogen without the direct interaction with other organisms. Without limitation said free living nitrogen fixing bacteria include the members of the genera *Azotobacter, Beijerinckia, Klebsiella, Cyanobacteria* (classified as aerobic organisms) and the members of the genera *Clostridium, Desulfovibrio* and the named Purple sulphur bacteria, Purple non-sulphur bacteria and Green sulphur bacteria. Associative nitrogen fixing bacteria are those prokaryotic organisms that are able to form close associations with several members of the *Poaceae* (grasses). These bacteria fix appreciable amounts of nitrogen within the rhizosphere of the host plants. Members of the genera *Azospirillum* are representative of associative nitrogen fixing bacteria. Symbiotic nitrogen fixation bacteria are those microorganisms which fix nitrogen symbiotically by partening with a host plant. The plant provides sugars from photosynthesis that are utilized by the nitrogen fixing microorganism for the energy it needs for nitrogen fixation. Members of the genera *Rhizobia* are representative of associative nitrogen fixing bacteria.

Also disclosed is an oxygen-sensitive protein from *Azotobacter vinelandii* (*A. vinelandii*). The term "is from" or "isolated from" means that said polynucleotide or encoded polypeptide is substantially separated or purified from other nucleic acid or encoded polypeptide in the cell of the organism in which the nucleic acid or encoded polypeptide naturally occurs. The term isolated thus encompasses nucleic acid purified by standard purification methods for nucleic acids or encoded polypeptide. The term also embraces nucleic acids or encoded polypeptide prepared by recombinant expression in a host cell as well as chemically synthesized nucleic acids or encoded polypeptide thereof.

The oxygen-sensitive protein is NifH. The term "NifH or dinitrogenase reductase or iron-protein or nitrogenase component II" refers to a component of the nitrogenase system which works in concert with NifDK (also known as Molybdenum-iron protein, dinitrogenase, or nitrogenase component I) to effect nitrogen reduction. NifH (accession number P00459, release of January 23, 2007) is a 60 kDa homodimer of the product of the *nifH* gene, with two-fold symmetry which contain sites for Mg²⁺, ATP binding and hydrolysis at the dimer interface within each subunit and a single [Fe₄-S₄] cluster at the interface of both subunits. The [Fe₄-S₄] cluster of NifH is coordinated by two cysteine residues from each subunit. Binding and hydrolysis of Mg²⁺ and ATP causes changes in NifH conformation and is coupled to electron transfer from the [Fe₄-S₄] cluster to the NifDK component.

The polynucleotide of the invention encodes a fusion protein as mentioned above which, in addition to the NifH protein, also comprises a mitochondrial targeting peptide. The term "mitochondrial targeting peptide or mitochondrial targeting signal (MTS) or mitochondrial localization signal (MLS)" refers to a 10-60 amino acid long peptide that directs a target protein to the mitochondria. It consists of an alternating pattern of hydrophobic and positively charged amino acids to form what is called amphipathic helix. Mitochondrial targeting signal can contain additional signals that subsequently target the protein to different regions of the mitochondria, such as the mitochondrial matrix. In a preferred embodiment, mitochondrial targeting peptide is N-terminal (amino terminus) to the NifH protein. Suitable mitochondrial targeting peptides that can be used in the context of the present invention include, without limitation, peptides having the general structure as defined by von Heijne (EMBO J., 1986, 5: 1335-1342) or by Roise and Schatz (J.Biol.Chem., 1988, 263: 4509-4511). Non limiting examples of mitochondrial targeting peptides are the mitochondrial targeting peptides defined in Table I of von Heijne (*supra*.) as well as mitochondrial targeting peptides of a mitochondrial polypeptide selected from the group consisting of human cytochrome c oxidase subunit VIII, the P1 isoform of subunit c of human ATP synthase, aldehyde dehydrogenase targeting sequence, Glutaredoxin 5, Pyruvate dehydrogenase, Peptidyl-prolyl isomerase, Acetyltransferase, Isocitrate dehydrogenase, cytochrome oxidase, and the subunits of the FA portion of ATP synthase. In a preferred embodiment, the mitochondrial targeting peptide is the mitochondrial targeting peptide of *Saccharomyces cerevisiae* (*S. cerevisiae*) superoxide dismutase (SOD) (SEQ ID NO: 1).

It may be useful in some embodiments of this invention to use multiple tandem copies of a chosen mitochondrial targeting peptide. The coding sequence for a duplicated o multiplied targeting peptide may be obtained through genetic engineering from an existing mitochondrial targeting peptide. The amount of mitochondrially-targeted peptide can be measured by cellular fractionation, followed by, for example, quantitative immunoblot analysis. Thus, in the present invention mitochondrial targeting peptide encompass one or more copies of one amino acid peptide that directs a target protein to the mitochondria. In a preferred embodiment, the mitochondrial targeting peptide comprises two copies of a chosen mitochondrial targeting peptide. In another embodiment, the mitochondrial targeting peptide comprises three copies of a chosen mitochondrial targeting peptide. In another embodiment, the mitochondrial targeting peptide comprises four copies or more of a chosen mitochondrial targeting peptide.

In a particular embodiment, the mitochondrial targeting peptide comprises a two tandem copies of the mitochondrial targeting peptide of *Saccharomyces cerevisiae* (*S. cerevisiae*) superoxide dismutase (SOD) (SEQ ID NO: 2).

In a particular embodiment, the polynucleotide of the invention encodes a fusion protein which further comprises at least one peptide tag adequate for detection or purification of fusion protein bound to the C-terminal or N-terminal domain of said fusion protein. In a preferred embodiment, said tag is N-terminal to the NifH protein. Said tag is generally a peptide or amino acid sequence which can be used in the isolation or purification of said fusion protein. Thus, said tag is capable of binding to one or more ligands, for example, one or more ligands of an affinity matrix such as a chromatography support or bead with high affinity. The skilled person will understand that the tag is located in the fusion protein at a location which does not result in the removal of the tag from the NifH protein once the mitochondrial targeting signal is cleaved off after import into the mitochondria. Moreover, the tag has to be located so that it does not interfere with the mitochondria import machinery. Thus, in a preferred embodiment, the polynucleotide of the invention encodes a fusion protein that comprises, in the N- to C-terminal order, an N-terminal mitochondrial targeting peptide, the detection/purification tag and the NifH protein. In other embodiment, the polynucleotide of the invention encodes a fusion protein that comprises, in the N- to C-terminal order, an N-terminal mitochondrial targeting peptide, the NifH protein and the detection/purification tag.

An example of said tag is a histidine tag (His-tag or HT), such as a tag comprising several residues of histidine (for example 6 residues [His6 or H6]; 8 residues [His8 or H8]), which can bind to a column of nickel (Ni²⁺) or cobalt (Co²⁺) with high affinity. His-tag has the desirable feature that it can bind its ligands under conditions that are denaturing to most proteins and disruptive to most protein-protein interactions. Thus, it can be used to remove the bait protein tagged with H6 following the disruption of protein-protein interactions with which the bait has participated.

Additional illustrative, non-limitative, examples of tags useful for detecting, isolating or purifying a fusion protein include fluoresecent tags such as fluorescein, resourfin and derivatives thereof, Arg-tag, FLAG-tag, Strep-tag, an epitope capable of being recognized by an antibody, such as c-myc-tag (recognized by an anti-c-myc antibody), SBP-tag, S-tag, calmodulin binding peptide, cellulose binding domain, chitin binding domain, glutathione S-transferase-tag, maltose binding protein, NusA, TrxA, DsbA, Avi-tag, etc. (Terpe K., Appl. Microbiol. Biotechnol. (2003), 60:523-525), an amino acid sequence such as Ala-His-Gly-His-Arg-Pro (SEQ ID NO: 3); Pro-Ile-His-Asp-His-Asp-His-Pro-His-Leu-Val-Ile-His-Ser (SEQ ID NO: 4); Gly-Met-Thr-Cys-X-X-Cys (SEQ ID NO: 5); β-galactosidase and the like.

In another embodiment, the fusion protein comprising the oxygen sensitive NifH protein, and a mitochondrial targeting peptide, preferably the mitochondrial targeting peptide of SOD also comprises a fluorescent protein. By "fluorescent protein" is meant any protein capable of emitting light when excited with appropriate electromagnetic radiation/light (i.e. light of an appropriate wavelength). The fluorescent protein will absorb energy of a specific wavelength and re-emit energy at a different (but equally specific) wavelength. The fluorescent protein can be N or C terminus to the oxygen sensitive protein. Fluorescent proteins that can be used include biological and chemical fluorophores. Exemplary biological fluorophores comprise T-sapphire, Cerulean, mCFPm, CyPet, EGFP, PA-EGFP, Emerald, EYFP, Venus, mCitrine, mKO1 (monomeric Kusabira orange 1) mOrange, DSRed, JRed, mStrawberry, mCherry, PA-mCherry, mRuby, Tomato, mPlum, mKate, mKatushka, Kaede, Halotag, and superecliptic fluorine. Exemplary chemical fluorophores comprise Alexafluor, Rhodamine, BODIPY, Tetramethylrhodamine, Cyanin dyes, Fluorescein, Quantum dots, IR dyes, FM dyes, ATTO dye. In another embodiment, the detection tag is a tetracysteine motif. As uses herein, "tetracysteine motif' refers to a short amino acid sequence containing four cysteines (CCXXCC) (SEQ ID NO: 6) encoded at the N or C terminal of the NifH protein which binds to biarsenical dyes, ReAsH (red fluorescent) and FIAsh (green fluorescent), with high specificity even in live cells. FlAsH is a fluorescein derivative, modified to contain two arsenic atoms at a set distance from each other. ReAsH is based on resorufin and has been similarly modified.

In a preferred embodiment, the polynucleotide according to the invention comprises SEQ ID NO:7.

The skilled person will understand that it may be desirable that fusion protein further comprises a flexible peptide that binds the oxygen sensitive NifH protein, and the purification/detection tag.

As used herein, the term "flexible peptide", "spacer peptide" or "linker peptide" refers to a peptide that covalently binds the NifH protein and the mitochondrial targeting peptide, which is not part of neither the NifH protein nor the mitochondrial targeting peptide, allowing movement of one with respect to the other, without causing a substantial detrimental effect on the function of either the protein or the moiety. In a preferred embodiment, said flexible peptide binds the NifH protein and the mitochondrial targeting peptide substantially without causing a detrimental effect on the function of neither the NifH protein nor the mitochondrial targeting peptide. It is not necessary that the NifH protein and the mitochondrial targeting peptide are arranged in that order and, in this case, the invention contemplates fusion proteins in which the NifH protein is located at amino-terminal position relative to the mitochondrial targeting peptide, and wherein the NifH protein is located at carboxyl-terminal position relative to the cell penetrating peptide.

The flexible peptide comprises at least one amino acid, at least two amino acids, at least three amino acids, at least four amino acids, at least five amino acids, at least six amino acids, at least seven amino acids, at least eight amino acids, at least nine amino acids, the least 10 amino acids, at least 12 amino acids, at least 14 amino acids, at least 16 amino acids, at least 18 amino acids, at least 20 amino acids, at least 25 amino acids, at least 30 amino acids, at least 35 amino acids, at least 40 amino acids, the least 45 amino acids, at least 50 amino acids, at least 60 amino acids, at least 70 amino acids, at least 80 amino acids, at least 90 amino acids, or about 100 amino acids. In some embodiments the flexible peptide will permit the movement of one protein with respect to the other in order to increase solubility of the protein and/or to improve its CPP activity. If desired, the flexible peptide can encompass either repetitions of poly-glycine or combinations of glycine, proline and alanine residues.

In a preferred embodiment, the sequences which encode the fusion protein of the polynucleotide of the invention are codon optimized for expression in a eukaryotic cell. The term "codon optimized", as used herein, refers to the alteration of codons in nucleic acid molecules to reflect the typical codon usage of the host organism without altering the polypeptide encoded by the DNA, to improve expression. There are several methods and software tools known in the art for codon optimization. See Narum D, et al., Infect. Immun. 2001; 69(12):7250-7253), Outchkourov N, et al., Protein Expr. Purif. 2002; 24(1):18-24, Feng L, et al., Biochemistry 2000; 39(50):15399-15409, and Humphreys D, et al., Protein Expr. Purif. 2000; 20(2):252-264.

In a still more preferred embodiment, said codon optimization is for expression in yeast.

In a still more preferred embodiment, the polynucleotide of the invention comprises a nucleotide encoding a fusion protein which comprises the oxygen sensitive NifH protein and a mitochondrial targeting peptide as described above, which are operatively linked to suitable transcriptional or translational regulatory elements. The transcriptional or translational regulatory elements can be derived from, for example, mammalian, microbial, viral, or insect genes. A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences, as described in detail below. Such regulatory elements can include an operator sequence to control transcription. The ability to replicate in a host, usually conferred by an origin of replication, and a selection gene to facilitate recognition of transformants can additionally be incorporated. DNA regions are operatively linked when they are functionally related to each other. For example, DNA for a signal peptide is operatively linked to DNA for a polypeptide if it is expressed as a precursor which participates in the secretion of the polypeptide; a promoter is operatively linked to a coding sequence if it controls the transcription of the sequence; or a ribosome binding site is operatively linked to a coding sequence if it is positioned so as to permit translation. The regulatory sequences useful for the present invention can be nuclear promoter sequences or, alternatively, enhancer sequences and/or regulatory sequences which increase the expression of the nucleotide sequence, suppressor sequences, transcriptional start sites, transcriptional stops sites, polyadenilation sites and the like. A great number of expression control sequences are known in the art and may be utilized according to the present invention. In the case of eukaryotic cells they comprise normally promoters ensuring initiation of transcription and optionally poly-A signals ensuring termination of transcription and stabilization of the transcript, for example, those of the 35S RNA from Cauliflower Mosaic Virus (CaMV). Others promoters commonly used are the Figwort Mosaic virus promoter, the polyubiquitin promoter and the actin promoter for ubiquitous expression. Possible regulatory elements permitting expression in eukaryotic host cells comprise e.g.SV40 promoter, Rous Sarcoma virus promoter, CMV enhancer, SV40 enhancer. The regulatory sequences useful for the present invention also encompass eukaryotic translational enhancers such as the CAMV omega sequences or the inclusion of introns which can increase the expression level by up to 100-fold (Maiti et al., 1997, Transgenic Research 6: 143-156). The promoter can be constitutive or inducible. If the constant expression of the polynucleotide is desired, then a constitutive promoter is used. An "inducible" promoter is used when is desired a regulated expression of the polynucleotide depending on physiological or developmental conditions. Typical promoters suitable for expression in yeast cells such include, but are not limited to:
- Constitutive promoters such as, for example, the alcohol dehydrogenase (ADH1) promoter, the 1-α elongation factor (TEF) promoter and the promoter of the gene which encodes triose phosphate isomerase (TPI), the glyceraldehyde 3-phosphate dehydrogenase (GPD) promoter and the 3-phosphoglycerate kinase (GPK) promoter, the MRP7 promoter and the alcohol oxidase (AOX1) promoter.
- Inducible promoters such as, for example, the metallothionein (CUP1) promoter, the expression of which is regulated by means of adding copper to the culture medium, the promoter of the gene which encodes the FUS1 gene or the FUS2 gene, the expression of which is activated in the presence of pheromones (the α factor) as described in US5063154, the TET promoter, the expression of which is regulated in the presence of tetracyclines, the GAL1-10, GALL, GALS promoters which are activated in the presence of galactose, the VP16-ER promoter, inducible by estrogens, and the phosphatase (PH05) promoter the expression of which is activated in the presence of phosphate and the HSP150 heat shock protein promoter, the expression of which is activated at a high temperature.
- Repressible promoters such as, for example, the *S.cerevisiae* enolase (ENO-1) gene promoter, the expression of which can be repressed when the microorganism is grown in a non-fermentable carbon source, as well as promoters the expression of which is subject to glucose repression such that the expression will be repressed when part of the lactose has been hydrolyzed and the concentration of glucose in the medium starts to increase, the *S. cerevisiae* glyceraldehyde-3-phosphate dehydrogenase (ADH2/GAP) promoter and the galactokinase (GAL 1) promoter.

Different promoters are preferably used in the two constructs such that the expression of the α-galactosidase and of the heterologous protein can be regulated independently. Preferably, in those cases in which the heterologous protein is suspected of being toxic to the host cell, the promoter used to regulate its expression is advisably an inducible promoter such that the expression of the protein of interest can be delayed until sufficient biomass levels have been achieved.

In another embodiment, the polynucleotide of the invention which encodes the NifH protein, preferably form *A. vinelandii*, and a mitochondrial targeting peptide N-terminal to the NifH protein, further comprises a nucleotide sequence which encodes a fusion protein comprising the NifM protein and a second mitochondrial targeting peptide.

The term "NifM" as used herein refers to the *nifM* gene and the NifM protein from *A vinelandii*. NifM protein (accession number P14890, released on April 1, 1990) is a peptidyl-prolyl cis-trans isomerase which is required for the maturation and activation of the Fe protein of the nitrogenase (Rubio and Ludden, 2005, J. of Bact., 187, 2: 405-414).

The fusion protein comprising NifM also comprises a second mitochondrial targeting peptide. The term "mitochondrial targeting peptide" and examples thereof suitable for using in this invention have been defined above and equally apply to this embodiment of the invention. Said mitochondrial targeting peptide is preferably located N-terminal to the NifM protein. The second mitochondrial targeting sequence may be the same or different as the first mitochondrial targeting sequence. In a preferred embodiment, the second mitochondrial targeting peptide is the mitochondrial targeting peptide of *S. cerevisiae* superoxide dismutase (SOD).

The order of the regions within the polynucleotide of the invention is not particularly limiting. Thus, in one embodiment, the region encoding the fusion protein comprising NifH and the mitochondrial targeting signal is located 5' with respect to the fusion protein comprising NifM and the second mitochondrial targeting signal

The skilled person will understand that it may be desirable that fusion protein comprising the NifM protein, and a second mitochondrial targeting peptide, preferably the mitochondrial targeting peptide of SOD further comprises a flexible peptide that binds the NifM and the fluorescent group. The term "flexible peptide" has been defined above and equally applies for this embodiment of the invention. If desired, that fusion protein which comprises the NifH protein, the mitochondrial targeting peptide N-terminal to said NifH protein and the fluorescent group, further comprises a tetracysteine motif. The term "tetracysteine motif' has been described for the fusion protein comprising the NifH protein and equally applies for this embodiment of the invention.

In a particular embodiment, said fusion protein comprising the NifM protein, comprises at least one peptide tag adequate for detection or purification of fusion-protein bound to the C-terminal or N-terminal domain of said fusion protein or variant thereof. In a preferred embodiment, said tag is N-terminal to the NifM protein. Said tag is generally a peptide or amino acid sequence which can be used in the isolation or purification of said fusion protein. Thus, said tag is capable of binding to one or more ligands, for example, one or more ligands of an affinity matrix such as a chromatography support or bead with high affinity. An example of said tag is a flag tag (Flag-tag or flag-octapeptide) which is recognized by some commercially available antibodies (e.g. M1/E11, M2). The flag tag's structure has been optimized for compatibility with the proteins it is attached to, in that it is more hydrophilic than other common epitope tags and therefore less likely to denature or inactivate proteins to which it is appended. In addition, N-terminal flag tags can be removed readily from proteins once they have been isolated, by treatment with the specific protease, enterokinase Additional illustrative, non-limitative, examples of tags useful for isolating or purifying a fusion protein have been described above and equally apply to this embodiment of the invention.

In a preferred embodiment, the sequences which encode the fusion protein comprising NifM protein have been codon optimized for expression in a eukaryotic cell. In a still more preferred embodiment, the codon optimization is for expression in yeast. The term "codon optimization" as well as typical promoters used for expression in yeast have been explained previously and equally apply to this embodiment of the invention.

In a preferred embodiment, the polynucleotide of the invention which encodes a fusion protein which comprises the NifH protein, preferably from *A. vinelandii*, and a mitochondrial targeting peptide N-terminal to the NifH protein and a nucleotide sequence which encodes a fusion protein comprising the NifM protein and a second mitochondrial targeting peptide wherein said mitochondrial targeting peptide is N-terminal to NifM further comprises suitable transcriptional or translational regulatory elements. Examples of suitable transcriptional and translational regulatory elements have been described above and equally apply to this embodiment of the invention.

In a preferred embodiment, the region within the polynucleotide of the invention which encodes a fusion protein which comprises the NifH protein, preferably from *A. vinelandii*, and a mitochondrial targeting peptide N-terminal and the region within the polynucleotide of the invention which encode the fusion protein comprising the NifM protein and a second mitochondrial targeting signal are under the control of a single transcriptional regulatory signal, so that both fusion proteins are encoded by a single mRNA. In this case, the polynucleotide of the invention may further comprise an internal ribosomal entry site (IRES) 5' to the region encoding the fusion protein which is located downstream in the polynucleotide of the invention. In another embodiment, the region within the polynucleotide of the invention which encodes a fusion protein which comprises the NifH protein, preferably from *A. vinelandii*, and a mitochondrial targeting peptide N-terminal and the region within the polynucleotide of the invention which encode the fusion protein comprising the NifM protein and a second mitochondrial targeting signal comprise independent transcriptional regulatory regions.

In another aspect, the invention relates to the use of a polynucleotide according to the invention for expressing a NifH protein.

### Expression vectors and cells of the invention

In another aspect, the invention refers to an expression vector comprising the polynucleotide of the invention.

The term "expression vector" refers to a replicative DNA construct used for expressing DNA which encodes the polypeptide of the invention and which includes a transcriptional unit comprising the assembly of (1) genetic element(s) which play a regulatory role in gene expression, for example promoters, operators or enhancers, operatively bound to (2) a DNA sequence encoding the polypeptide of the invention which is transcribed into messenger RNA and translated into protein and (3) suitable sequences to initiate and to terminate the transcription and translation. Preferably, in this case in which is desirable the expression of this two heterologous genes, *nifH* and *nifM*, an expression vector comprising GAL1-GAL10 divergent promoters can be used allowing the co-expression of both proteins.

The vectors that can be used in the context of the present invention normally include a genetic marker, a replication origin in bacteria or yeasts, multiple cloning sites, and a genetic marker. The genetic marker is usually a gene conferring resistance to an antibiotic or alternatively, an auxotrophic marker in the case of yeasts.

The yeast vectors suitable for the present invention can be based on the following types of plasmids:
- Multicopy autonomous plasmids: These plasmids contain sequences which allow generating multiple copies of said vectors. These sequences can be the so-called 2µ such as the one which appears in episomal plasmids (YEp or yeast episomal plasmids) or ARS-type sequences such as those which appear in replication plasmids (YRps or yeast replication plasmids). Examples of vectors based on plasmids of this type are p426GPD, p416GPD, p426TEF, p423GPD, p425GPD, p424GPD or p426GAL, YEp24 and YEplac.
- Single copy autonomous plasmids: Plasmids which contain the autonomous replication sequence ARS1 and a centromere sequence (CEN4). Plasmids of this type include the centromere plasmids (YCps or yeast centromere plasmids).
- Integrating plasmids: Plasmids which are capable of being integrated into the host cell genome. Plasmids of this type include integrating plasmids (YIPs or yeast integrating plasmids). Examples of vectors based on plasmids of this type are pRS303, pRS304, pRS305 or pRS306 and the like.

Generally, all the vectors mentioned by Sikorski ("Extrachromosomal cloning vectors of Saccharomyces cerevisiae", in Plasmid, A Practical Approach, Ed. K. G. Hardy, IRL Press, 1993) and by Ausubel et al. ("Yeast Cloning Vectors and Genes" Current Protocols in Molecular Biology, Section II, Unit 13.4, 1994) are useful in the context of the present invention.

In another aspect, the invention relates to a eukaryotic cell containing the polynucleotide according to the invention or the expression vector containing the polynucleotide according to the invention.

In another preferred embodiment, said eukaryotic cell grows under aerobic conditions. The term "aerobic conditions" as used herein refers to an oxygenated environment. Eukaryotic cells which grow under aerobic conditions include: facultative anaerobes organisms, which include organisms which can use oxygen, but also have anaerobic methods of energy production; obligate aerobes organisms, which require oxygen for aerobic cellular respiration wherein said organisms use oxygen to oxidize substrates (i.e. sugars and fats) in order to obtain energy; microaerophiles organisms, which include organisms that may use oxygen but at low concentrations; and aerotolerant organisms, which are those organisms that can survive in the presence of oxygen, but they are anaerobic because they do not use oxygen as a terminal electron acceptor.

In a still more preferred embodiment, said eukaryotic cell is a yeast cell. Yeast cells belong to facultative anaerobic organisms and they obtain energy (ATP) by aerobic respiration if oxygen is present but they are also capable of switching to fermentation. Yeast is understood as any eukaryotic organism belonging to the ascomycetes type which includes the organisms generally known as yeasts as well as those generally known as filamentous fungi. The yeasts and filamentous fungi include *Pichia* sp (for example, *P. pastoris, P. finlandica, P. trehalophila, P. koclamae, P. membranaefaciens, P. minuta, P. opuntiae, P. thermotolerans, P. salictaria, P. guercuum, P. pijperi, P. stiptis, P. methanolica), Saccharomyces (S. cerevisiae), Schizosaccharomyces pombe, Kluyveromyces* (for example, *K. lactis, K. fragilis, K. bulgaricus, K. wickeramii, K. waltii, K. drosophilarum, K. thernotolerans, and K. marxianus, K. yarrowia), Trichoderma reesia, Neurospora crassa, Schwanniomyces, Schwanniomyces occidentalis, Penicillium, Totypocladium, Aspergillus (*for example, *A. nidulans, A. niger, A. oryzae), Hansenula polymorpha, Candida, Kloeckera, Torulopsis, and Rhodotorula, Hansenula, Kluyveromyces sp. (*for example, *Kluyveromyces_lactis), Candida albicans, Aspergillus sp (*for example, *Aspergillus nidulans, Aspergillum niger, Aspergillus oryzae), Trichoderma reesei, Chrysosporium luchiowense, Fusarium sp.* (for example, *Fusarium gramineum, Fusarium venenatum), Physcomitrella patens.*

Virtually any yeast can be considered in the present invention; however, in a particular embodiment, said yeast is yeast from the *Saccharomyces* genus, such as *S. cerevisiae*.

In another aspect, the invention relates to the use of an expression vector according to the invention or of a cell according to the invention for expressing aNifH protein.

### Method to express NifH in a eukaryotic cell

In another aspect, the invention relates to a method for expressing NifHin a eukaryotic cell comprising the steps of:
i) introducing into said cell a polynucleotide according to the invention or a vector comprising said polynucleotide,
ii) growing said cell under conditions allowing the expression of said oxygen-sensitive protein and, if desired;
iii) purifying said oxygen-sensitive protein under anaerobic conditions.

The terms "oxygen sensitive protein" and "eukaryotic cell" have been previously defined and equally apply to his aspect of the invention.

In the first step the method to express aNifH protein in a eukaryotic cell (thereinafter, first method of the invention) comprises introducing into said eukaryotic cell the polynucleotide or the vector according to the invention.

The term "introducing into eukaryotic cell the polynucleotide of the invention or introducing into eukaryotic cell the expression vector of the invention" refers to a process for delivering said nucleic acid into yeast cell. Methods suitable for introducing a DNA molecule into a yeast cell include but are not limited to:
- Transformation of spheroplasts which entails removing the cell wall of the yeast and contacting the spheroplasts with the plasmid in the presence of PEG.
- Transformation with Li⁺, which entails the treatment of yeast cells with monovalent alkaline cations (Na⁺, K⁺, Rb⁺, Cs⁺ and Li⁺) in combination with PEG to stimulate DNA uptake by the intact cells.
- Gen gun which entails bombarding cells with microprojectiles coated with the exogenous DNA.
- Electroporation, which entails administering electrical pulses to the yeasts which results in the opening of pores in the membrane of the spheroplasts and intact yeast cells.

Transformants are grown in an appropriate nutrient medium, and, where appropriate, maintained under selective pressure to insure retention of endogenous DNA. Where expression is inducible, growth can be permitted of the yeast host to yield a high density of cells, and then expression is induced. The non-yeast protein can be harvested by any conventional means, and purified by chromatography, electrophoresis, dialysis, solvent-solvent extraction, and the like.

The second step of the first method of the invention comprises growing said transformed cells under conditions allowing the expression of the NifH protein according to the invention. Said conditions suitable for the expression of the NifH protein according to the invention include conditions to be those that allow an optimal growth of the yeast cell and those that allow the protein expression in said cells. Conditions for optimizing the culture of yeast cells and the expression of said heterologous protein will depend on the promoter which regulates the expression of the heterologous gene.

In a preferred embodiment, the transformed cell contains a polynucleotide encoding the NifH protein and a mitochondrial targeting peptide, wherein said mitochondrial targeting peptide is N-terminal to the NifH protein and, preferably SOD from *S. cerevisiae,* operatively linked to suitable transcriptional or translational regulatory elements. In a still more preferred embodiment, the expression of *nifH* gene is directed from GAL1 promoter. Optimal conditions for cell grown and NifH expression under GAL1 promoter are for example, those wherein transformants are grown under aerobic conditions to saturation in minimal selective medium containing high levels of glucose, such as 2% glucose at 30°C. Once the glucose is consumed 2% galactose can be added to the cell culture to induce NifH protein expression. After induction of NifH expression, cells can be cultured for 24-72 hours allowing maximum protein production.

In another preferred embodiment, the transformed cell of the invention contains the polynucleotide of the invention which encodes a fusion protein which comprises the NifH protein, and a mitochondrial targeting peptide, preferably SOD from *S. cerevisiae,* N-terminal to the NifH protein, further comprises a nucleotide sequence which encodes a fusion protein comprising the NifM protein and a mitochondrial targeting peptide, preferably SOD from *S. cerevisiae,* wherein said mitochondrial targeting peptide is N-terminal to NifM, operatively linked to suitable transcriptional or translational regulatory elements. Preferably, in this case in which is desirable the expression of this two heterologous genes, *nifH* and *nifM*, an expression vector comprising GAL1-GAL10 divergent promoters can be used allowing the co-expression of both proteins. Optimal conditions for cell grown and NifH and NifM expression are those conditions explained above.

Taking into account that the first and the second step of the first method of the invention are intended to express and purify aNifH protein, it is desirable to test if anaerobic conditions have been maintained during said protein induction and purification. The fusion protein of the invention (or the polynucleotide which codes said fusion protein) is expressed in the mitochondria wherein anaerobiosis conditions occur. Thus, any test which allows the determination of mitochondrial activity would be useful to determine the lack of oxygen stress conditions during galactose induction. If desired, aconitase enzyme activity can be measured (see for example Kennedy et al., 1983, J. Biol. Chem, 258: 11098-11105).

The expression of the fusion protein during the second step of the first method of the invention can be monitored by methods well known in the art such western blot or immunofluorescence. Virtually any conventional method can be used within the frame of the invention to detect, and if desired, to quantify the NifH protein. By way of a nonlimiting illustration, the expression levels can be determined by means of antibodies with the capacity for binding specifically to the assayed protein (or to fragments thereof containing the antigenic determinants) and subsequent quantification of the resulting antigen-antibody complexes.

There is a wide variety of known tests that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), immunofluorescence. Other forms of detecting and quantifying protein include, for instance, affinity chromatography techniques or ligand-binding assays. Since the mitochondrial targeting signal is cleaved upon entry of the fusion protein into the mitochondria, it is also possible to monitor the accumulation of the protein in the mitochondria by detecting the accumulation of the processed fusion protein using any technique which allows detecting the change in molecular weight that occurs following removal of the mitochondrial targeting signal. In a preferred embodiment, the detection of the mature protein is carried out by western blot of whole cell protein extracts using an antibody specific against the oxygen-senstive NifH protein or against any protein tag forming part of the fusion protein.

In a preferred embodiment of the invention, the determination of the levels of the fusion proteins is performed by quantitative immunofluorescence. Immunofluorescence (IF) is a technique based on fluorescent microscopy utilized primarily for testing biological samples. This technique uses the specificity of antibodies to their antigen to target fluorescent dyes to particular biomolecule targets within a cell, thus allowing the visualization of the distribution of the target molecule through the sample. IF can be used in cultured cell lines, or individual cells, and may be used to analyze the distribution of proteins, glycans, and small biological and non-biological molecules. In addition, IF could also be used in combination with other, non-antibody methods of fluorescent staining, such as, for example, DAPI to label DNA. More than one antibody can be used at the same time in order to detect, for example more than one protein. Several microscope designs can be used for analysis of IF samples, such as the epifluorescence microscope and the confocal microscope. Various super-resolution microscope designs that are capable of much higher resolution may also be used. In a still more preferred embodiment, a double staining is carried out. In said double staining the oxygen sensitive NifH protein of the invention can be detected as well as the specific location of said protein in the cell. For example, yeast cells transfected with the polynucleotide or with the expression vector of the invention which encodes a fusion protein comprising the NifH protein, the SOD peptide and the GFP protein can be fixed with a solution of paraformaldehyde (2-4%) and permeabilized with 0.1 % TX-100. The mitochondrial localization of NifH can be confirmed by means of specifically stain. Mitochondria stain can be carried out by techniques well known in the art. Examples of said techniques include but are not limited to rhodamine 123 staining, tetramethylrosamine or mitotracker stain. In a preferred embodiment, mitochondrial are detected by mitrotracker stain. Briefly, this technique is based in using fluorescent dyes that stain mitochondria in live cells. Specific mitotracker probes are commercially available such as rosamine mitotracker dyes which include MitoTrackerR Orange CMTMRos, a derivative of tetramethylrosamine, and MitoTrackerR Red CMXRos (Invitrogen), a derivative of X-rosamine. Reduced MitoTrackerR dyes, MitoTrackerR Orange CM-H2TMRos and MitoTrackerR Red CM-H2XRos (Cat. no. M7513), which are derivatives of dihydrotetramethyl rosamine and dihydro-X-rosamine (Invitrogen). These reduced probes do not fluoresce until they enter live cells, where they are oxidized to the corresponding fluorescent mitochondrion-selective probe and then sequestered in the mitochondria.

If desired, the first method of the invention comprises an additional step which comprises the purification of said oxygen sensitive NifH protein. In order to preserve the activity of the fusion protein, it is recommended that the purification is carried out under anaerobic conditions. As used herein, the term "anaerobic conditions" refers to conditions wherein the oxygen concentration is below 1 ppm. In a preferred embodiment, the NifH is from *A. vinelandii*.

The purification of the NifH protein requires that the cells in which the protein is expressed are lysed. The skilled person will understand that the purification can be carried out by applying a single lysis step so that all biological membranes are disrupted, including the cell membrane and the mitochondrial membranes, thereby obtaining a whole cell lysate which can then be further processed in order to purify the NifH protein. Alternatively, the purification may be carried out in isolated mitochondria, which are first obtained from cells by a first lysis step aimed at lysing the cell membrane and, as the case may be, the cell wall. The isolated mitochondrial are then lysed in order to obtain a mitochondrial lysate from which the NifH is purified.

If desired, the NifH producing *S. cerevisiae* cells are first lysed under anaerobic conditions in order to isolate said protein. Cells can be resuspended in anaerobic lysis buffer containing: 50 mM sodium phosphate pH 8.0, 0.5 M NaCl, 10 mM imidazole, 1 mM phenylmethylsulfonyl fluoride (PMSF), 1 mM leupeptin, 2 mM sodium dithionite (DTH), 5 µg/ml DNaseI and preferably, any protease inhibitor in order to evoke protein degradation. Cells can be lysed in a French Press cell at 1500 lb/in². Cell-free extracts obtained after removing debris can be obtained by centrifugation at 17,000 rpm for 1 hour at 4°C under anaerobic conditions.

In order to isolate and purify the NifH protein according to the invention, any technique known in the art for protein purification under anaerobic conditions can be used (see for example, Curatti et al., 2006, Proc.Natl. Acad. Sci. USA, 103: 5297-5301; Christiansen et al. 1998, Biochemistry 37:12611-12623). In one embodiment, the fusion protein according to the invention is isolated using antibodies which are capable of specifically binding to either the NifH protein or, as the case may be, to the purification tag. Antibodies suitable for the immuno-isolation of the fusion protein include, without limitation, monoclonal antibodies, polyclonal antibodies or fragment thereof, Fv, Fab, Fab' and F(ab')2, scFv, diabodies, triabodies, tetrabodies and humanized antibodies.

In a preferred embodiment, in those embodiments wherein the fusion protein further comprises a tag, purification of the NifH protein is carried out by affinity chromatography using a reagent which specifically binds to said tag. In this case, the tag in the fusion protein and the reagent showing affinity for said tag act as first and second members of a binding pair, respectively.

The term "first member of a binding pair", as used herein, refers to a molecule which has affinity for and "binds" to another (hereinafter known as "second member of the binding pair") under certain conditions, referred to as "binding conditions". The first member of the binding pair is a peptide (protein) whereas the second member of the binding pair may be a of peptide or non-peptide nature.

The term "binding pair" does not involve any particular size or any other technical structural characteristic other than that said binding pair can interact and bind to the other member of the binding pair resulting in a conjugate wherein the first and second components are bound to each other by means of the specific interaction between the first and second member of a binding pair. The binding pair includes any type of immune interaction such as antigen/antibody, antigen/antibody fragment, hapten/anti-hapten as well as non-immune interactions such as avidin/biotin, avidin/biotinylated molecules, folic acid/folate-binding protein, hormone/hormone receptor, lectin/carbohydrate, lectin/molecule modified with carbohydrates, enzyme/enzyme substrate, enzyme/enzyme inhibitor, protein A/antibody, protein G/antibody, complementary nucleic acids (including sequences of DNA, RNA and peptide nucleic acids (PNA)), polynucleotide/polynucleotide-binding protein and the like.

As used in the present invention, the expression "specific binding" refers to the capacity of a first molecule to bind specifically to a second molecule by means of the existence of complementarity between the three-dimensional structures of the two molecules with a substantially higher affinity for non-specific binding such that the binding between said first and second molecule preferably takes place before the binding of any of said molecules with respect to the other molecules present in the reaction mixture. It is understood that there is high affinity in the binding of two molecules when the complex resulting from said binding has a dissociation constant (KD) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M or less than 10⁻¹⁵ M.

In a preferred embodiment, the NifH is expressed as fusion comprising a histidine tag, in which case the purification can be carried out using immobilized metal affinity chromatography (IMAC) This technique works by allowing proteins with affinity for metal ions (i.e. fusion protein comprising NifH fused to a histidine tag) to be retained in a column containing immobilized metal ions, such as cobalt, for the purification of histidine containing proteins or peptides. Eluted fractions can be then concentrated using a Vivaspin 500 concentrator (Sartorius) with cut-off pore size of 30 kDa as is shown in Example 3 of the present application.

### Method for in vitro reconstitution of active nitrogenase protein complex

The method for expression and recovery of active NifH according to the present invention allows obtaining amounts of NifH that can be used for reconstitution of active nitrogenase complex in the presence of the NifDK complex. Thus, in another aspect, the invention relates to a method for *in vitro* reconstitution of active nitrogenase protein complex comprising the step of:
i) introducing into a eukaryotic cell the polynucleotide of the invention or a vector according to the invention, wherein the cell further comprises a nucleotide sequence which encodes a fusion protein comprising the NifM protein and a mitochondrial targeting peptide wherein said second mitochondrial targeting peptide is N-terminal to NifM,
ii) growing said cell under conditions allowing the expression of NifH and NifM proteins
iii) purifying NifH protein from said cells under anaerobic conditions, and
iv) contacting said purified NifH of step iii) with a NifDK complex.

The term "protein complex" or "complex" as used herein, is related to a group of two or more associated polypeptide chains. Preferably, said polypeptides interact with each other at the same time and place. Protein complexes are a form of quaternary-structure. Proteins in protein complexes are linked by non-covalent protein-protein interactions, and different complexes have different degrees of stability over time. As used herein, the term "active reconstituted nitrogenase complex" refers to a protein complex that is formulated from individual, isolated recombinants which retains substantially all the functional activity of the native nitrogenase protein complex from which the peptides are isolated. "Nitrogenase activity" refers to the reaction that the nitrogenase complex performs which is the enzymatic reduction of dinitrogen to ammonia:

N₂ + 8H⁺ + 8e⁻ + 16 ATP → 2 NH₃ + H₂ + 16ADP + 16Pᵢ

Thus, the term "nitrogenase complex" refers to a protein complex which comprises the nitrogenase reductase component or iron-protein or nitrogenase component II or NifH and the molibdenum-iron protein or nitrogenase component I or NifDK. Said complex catalyzes other reactions which involve the reduction of triple or double bound substrates, such as acetylene reduction, nitrous oxide reduction, azide reduction, cyanide reduction, carbonil sulfide reduction, carbon dioxide reduction, thiocyanate reduction or cyanate reduction.

In a preferred embodiment, said active reconstituted nitrogenase complex has a nitrogenase activity of at least 5%, 10%, 15%, 20%, 25%, 30%, 25%, 30%, 35%, 40%, 45%, 50%, 55%, 60%, 65%, 70%, 75%, 80%, 85%, 90%, 95%, 100% with respect to said nitrogenase activity of the native nitrogenase complex isolated from *A. vinelandii*; preferably at least 90%; preferably at least 95%; more preferably at least 96%; still more preferably at least 97%; preferably at least 98%; more preferably at least 99%; still more preferably at least 100%.

The first step of the method for *in vitro* reconstitution of active nitrogenase protein complex (hereinafter, the second method of the invention) comprises introducing in the eukaryotic cell the polynucleotide of the invention which encodes a fusion protein which comprises the NifH protein, and a mitochondrial targeting peptide, preferably SOD from *S. cerevisiae,* N-terminal to the NifH protein. The cell wherein the polynucleotide is introduced further comprises a nucleotide sequence which encodes a fusion protein comprising the NifM protein and a mitochondrial targeting peptide, preferably SOD from *S. cerevisiae,* wherein said mitochondrial targeting peptide is N-terminal to NifM, operatively linked to suitable transcriptional or translational regulatory elements.

The terms "oxygen-sensitive protein", "mitochondrial targeting peptide", "NifH" and "NifM" as well as their preferred embodiments have been defined previously and equally apply to this aspect of the invention.

The term "introducing into eukaryotic cell the polynucleotide of the invention or introducing into eukaryotic cell the expression vector of the invention" has also been defined related to the method to express a NifH protein in a eukaryotic cell of the invention and equally applies to the second method of the invention.

In one embodiment, the polynucleotide according to the invention and encoding a fusion protein comprising NifH and the mitochondrial targeting signal is introduced into a cell that already comprises a nucleotide sequence which encodes a fusion protein comprising the NifM protein and a mitochondrial targeting peptide, preferably SOD from *S. cerevisiae,* wherein said mitochondrial targeting peptide is N-terminal to NifM, operatively linked to suitable transcriptional or translational regulatory elements. Said a nucleotide sequence which encodes a fusion protein comprising the NifM protein and a mitochondrial targeting peptide may be provided in a plasmid (extrachromosomally) or integrated in the genome of the host cell.

In another embodiment, the polynucleotide according to the invention and encoding a fusion protein comprising NifH and the mitochondrial targeting signal and the polynucleotide which encodes a fusion protein comprising the NifM protein and a mitochondrial targeting peptide are provided in a single polynucleotide.

The second step of the second method of the invention comprises growing said transformed cells under conditions allowing the expression of both proteins, NifH and NifM. Said conditions suitable for the expression of said proteins include conditions to be those that allow an optimal growth of the eukaryotic cell and those that allow the protein expression in said cells. Conditions for optimizing the culture of eukaryotic cells and the expression of said heterologous protein have been described above for the method to express a NifH protein in a eukaryotic cell according to the invention and equally applies for this aspect of the invention. Methods for monitoring whether sufficient amount of NifH has been produced are defined in the context of the first method of the invention and are equally applicable to the present method.

The method for *in vitro* reconstitution of active nitrogenase protein complex of the invention comprises a third step which comprises the purification of the NifH protein under anaerobic conditions. In a preferred embodiment, said protein is NifH from *A. vinelandii*. Methods and conditions for purification of NifH protein under anaerobic conditions have been detailed above in the first method of the invention and equally apply to the second method of the invention.

The fourth step of the second method of the invention comprises contacting the purified NifH protein of step (iii) with a NifDK complex. The term "NifDK complex" as used herein, refers to a heterotetrameric protein formed by two αβ dimers (α₂β₂) which carries one iron-molybdenum cofactor within the active site in each α-subunit. Said protein complex is the product of the *nifD* and *nifK* genes. In a preferred embodiment NifDK complex is isolated from *A. vinelandii*. The term "isolated from" has been defined above and equally applies to this aspect of the invention. Thus, in a preferred embodiment the *nifD* gene is of *A. vinelandii* origin. The *nifD* gene encodes the α subunit of the NifDK protein complex. The encoded protein sequence of *nifD* gene from *A. vinelandii* is available in Uniprot data base under accession number P07328 (January 23, 2007). The *nifK* gene encodes the β subunit of the NifDK protein complex. In a preferred embodiment, the *nifK* gene is of *A. vinelandii* origin. The encoded protein sequence of *nifK* gene from *A. vinelandii* is available in Uniprot data base under accession number P07329 (January 23, 2007).

Methods and conditions for growing *A. vinelandii* are well known in the art. Cells can be grown on limiting concentration of NH₄⁺ (100 µg as ammonium acetate) at 30°C. A. vinelandii is derepressed for nitrogenase in medium free of nitrogen source. Cells can be harvested at 2 hours after exhaustion of the ammonium. Once, the nitrogenase complex has been derepressed, said NifDK complex form *A. vinelandii* can be purified under anaerobic conditions by means of techniques well known in the skill in the art (Christiansen et al. 1998, Biochemistry 37:12611-12623). NifDK can be purified from cell-free extracts of *A. vinelandii* by affinity chromatography to a Co²⁺ resin under anaerobic conditions inside a glove box. In order to maintain anaerobic conditions buffers for protein purification and analysis are sparged with purified N₂ for 20-30 min. The *A. vinelandii* cell-free extracts can be prepared by osmotic shock followed by centrifugation at 30,000 × g for 1 h to remove cell debris. Cell-free extracts are loaded onto a 20-ml Co²⁺-affinity column equilibrated in 10 mM sodium phosphate, 1.8 mM potassium phosphate buffer (pH 7.3), 140 mM NaCl, 2.7 mM KCl, 10% glycerol. The column is washed with 200 ml of 50 mM Tris-HCl buffer (pH 7.9), 500 mM NaCl, 25 mM imidazole, and the NifDK protein complex is eluted from the column by applying 40 ml of 50 mM Tris-HCl buffer (pH 7.9), 150 mM NaCl, 300 mM imidazole. If desired, eluted NifDK can be concentrated by ultrafiltration through a YM10 membrane in an Amicon cell under a N₂ atmosphere and then subjected to a cycle of dilution-concentration in 50 mM Tris-HCl buffer (pH 7.9), 150 mM NaCl to remove the residual imidazole. A typical purification procedure yielded 100 mg of NifDK from 340 g of cell paste. Purity of NifDK protein complex can be estimated by means of SDS/PAGE analysis.

The term "contacting the purified NifH protein of step (iii) with a NifDK complex", as used herein refers to incubation of said purified NifH protein and said NifDK protein complex in conditions suitable for the interaction between said proteins; thereby leading to the formation of the nitrogenase protein complex of the invention.

The conditions under which the purified NifH with NifDK complex are carried out include a specific molar ratio between said NifH and NifDK components of the reaction. The term "molar ratio" as used herein refers to the ratio between the amounts in moles of any two compounds (i.e. NifH/NifDK) involved in a chemical reaction. In one embodiment, said molar ratio is related to the ratio between the amounts in moles of said two compounds wherein the maximum yield of the reaction is achieved. In a preferred embodiment, said molar ratio between NifH/NifDK is at least 1:100, 1:90, 1:80, 1:70, 1:60, 1:50, 1:40, 1:30, 1:20, 1:10, 1:9, 1:8, 1:7, 1:6, 1:5, 1:4, 1:3, 1:2, 1:1, 2:1, 4:1, 6:1, 8:1, 10:1, 20:1, 30:1, 40:1, 50:1, 60:1, 70:1, 80:1, 90:1, 100:1 or more.

The detection of said protein complex can be carried out by any method which allows the detection interaction among the NifH protein and the NifDK complex. Alternatively, the detection of the protein complex can be carried out by detecting the enzymatic activity of the resulting nitrogenase complex.

Methods suitable for detecting the existence of an interaction among the NifH protein and the NifDK complex include any method known in the art for detecting the protein-protein interaction including co-immunoprecipitation, affinity blotting, pull down, FRET and the like.

In the case that the formation of the complex containing NifH and NifDK protein is determined by measuring the nitrogenase activity, any method known in the art for detecting the enzymatic reduction of dinitrogen to ammonia wherein electrons are transferred from the NifH component to the NifDK protein complex can be used. Optimal conditions which allow nitrogenase activity are detailed in Example 6 of the present invention. Methods for determine if said nitrogenase complex is an active enzymatic complex are well known in the art and can be used (without limitation) in the present invention. If desired, the nitrogen fixation activity can be estimated by the acetyle reduction assay. Briefly, this technique is an indirect method which uses the ability of the nitrogenase complex to reduce triple bounded substrates. The nitrogenase enzyme reduces acetylene gas to ethylene. Both gases can be quantified using gas chromatography. Another way of measuring nitrogen fixation is directly by 15N₂ assimilation. The fixation and incorporation of 15 N₂ is measured by emission spectrophotometry or mass spectrometry. Another method to measure nitrogen fixation is by CHN analyses, Kjedahl digestion/oxidation, and high temperatures catalytic combustion techniques measure nitrogen accumulation into particle, dissolved or total organic matter.

### Kit of the invention

Additionally, the present invention relates to a kit useful to put into practice the invention disclosed herein.
i) a polynucleotide according to the invention or an expression vector according to the invention; and
ii) reagents suitable for expressing NifH protein in a eukaryotic cell and/or reagents to carry out the method for in vitro reconstitution of active nitrogenase protein complex in a eukaryotic cell according to the invention.

In the present invention a "kit" is understood as a product containing the different reagents and material to express NifH protein in a eukaryotic cell according to the method of the invention. The term kit also encompasses a product containing the different agents and material to *in vitro* reconstitution of an active nitrogenase protein complex in a eukaryotic cell according to the invention. Illustrative examples of reagents useful to carry out the methods of the invention are medium to keep cells, buffers, saline, etc. In a preferred embodiment, the kit according to the invention further comprises a nitrogenase substrate. As used herein the term "nitrogenase substrate" is understood as the reactant which is consumed during the reaction catalyzed by the nitrogenase enzyme. Examples of nitrogenase substrates are without limitation, nitrogen gas (N₂), nitrous oxide (N₂O), cyanure, carbon monoxide, methyl isocyanate, azide, acetylene, cyclopropane, cyanamide, diazirine. In a still more preferred embodiment, said nitrogenase substrate is acetylene. In another still more preferred embodiment, said nitrogenase substrate is nitrogen gas (N₂).

Another component which can be present in the kit is a packing which allows maintaining the agents properly stored. Suitable materials for preparing such packagings include glass, plastic (polyethylene, polypropylene, polycarbonate and the like), bottles, vials, paper, sachets and the like. The kit of the invention can additionally contain instructions for using the agents in the method to express NifH protein in a eukaryotic cell of the invention and/or instructions for using agents in the method to reconstitute in vitro an active nitrogenase protein complex. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain internet websites providing said instructions.

The invention is detailed below by means of the following examples which are merely illustrative and by no means limiting for the scope of the invention.

### EXAMPLES

### MATERIALS AND METHODS

**Strain growth and culture media.** *Saccharomyces cerevisiae* W303-1a (*MATa* {*leu2-3,112 trp1-1 can1-100 ura3-1 ade2-1 his3-11,15*} and derivative strains constructed herein were grown at 30°C and 200 rpm in YPD medium (1% yeast extract, 2% Bacto-Peptone, 2% glucose) or in minimal SD medium (7.7% yeast-nitrogen base and 2% glucose) supplemented with auxotrophic requirements (see Sherman, F. et al., 1986, Methods in yeast genetics, old Spring Harbor Laboratory, Cold Spring Harbor, NY).

*Escherichia coli* DH5α was used for storage and amplification of yeast expression vectors. *E.coli* strains were grown at 37°C in Luria-Bertani medium supplemented with ampicillin when necessary.

*Azotobacter vinelandii* DJ strain was cultivated in Burk's modified medium supplemented with 29 mM ammonium acetate at 30°C with shaking (200 rpm). Nitrogenase derepression was carried out after removing the ammonium acetate from the medium as described in Shah et al. (Shah et al, 1972, Biochim Biophys Acta, 256:498-511).

### Example 1

### DNA Constructs and generation of S. cerevisiae strains

*A. vinelandii nifH* and *nifM* genes were synthesized with codon optimization for *S. cerevisiae* (GenScript, USA). The N-terminal end of *nifH* carries DNA sequences encoding a duplicated superoxide dismutase mitochondrial leading signal from *S. cerevisiae* (*mlsSOD2*) and an eight-histidine tag. The *nifM* gene carries an N-terminal *mlsSOD2* and sequences encoding a Flag-tag. To determine NifM and NifH cellular localization, DNA constructs were synthesized which included N-terminal *mlsSOD2,* a tetracysteine motif *(cys₄),* and sequences encoding yEGFP or mKO, respectively. Synthetic constructs were cloned into the dual multicloning sites of pESC-His vector (Agilent Technologies) by using standard techniques.

*S. cerevisiae* W303-1A transformation was carried out according to Chen et al. (Chen et al., 1992 Biochim Biophys Acta, 1171:219-223). Strain GF1 carries *mlsSOD2-flag-nifM* under the control of GALlOp in pESC-His. Strain GF2 carries both GAL1p::*mlsSOD2-his₈-nifH* and GAL10p::*mlsSOD2-flag-nifM* in pESC-His. Strain GF3 carries GAL1p::*mlsSOD2-gfp-nifH-cys₄* in pESC-His. Strain GF4 carries GAL10p::*mlsSOD2-cys₄-nifM-mKO* in pESC-His.

### Example 2

### Induction of NifH and NifM expression

Inoculating cultures were prepared by growing *S. cerevisiae* at 30°C and 200 rpm in 1-liter flasks containing 500 ml of SD medium supplemented with auxotrophic requirements to an OD₆₀₀ of 0.6. For protein purification experiments, *S. cerevisiae* cells were cultivated in 4-liter batches of YPD medium in a 5-liter fermentor (Biostat B Plus, Sartorius) until glucose was consumed, at which time 2% galactose was added to induce NifH and NifM expression, and collected 72 h after galactose addition. Cells were collected at 4°C by centrifugation at 5,000 rpm for 5 min, and then frozen into liquid nitrogen.

### Example 3

### NifH purification

*S. cerevisiae* GF2 cells were resuspended in anaerobic lysis buffer containing: 50 mM sodium phosphate pH 8.0, 0.5 M NaCl, 10 mM imidazole, 1 mM phenylmethylsulfonyl fluoride (PMSF), 1 mM leupeptin, 2 mM sodium dithionite (DTH), 5 µg/ml DNaseI, and the yeast protease inhibitor cocktail (Sigma). Cells were lysed in a French Press cell at 1500 lb/in². Cell-free extracts were obtained after removing debris by centrifugation at 17,000 rpm for 1 hour at 4°C under anaerobic conditions.

NifH protein was purified by cobalt affinity chromatography under anaerobic conditions (<0.1 ppm O₂) inside a MBraun glove box. Cell-free extract was loaded at 0.2 ml/min onto a column filled with 20 ml Talon resin (Clontech), preequilibrated with lysis buffer, by using an AKTA Prime FPLC system (GE Healthcare). The column was then washed with wash buffer (50 mM sodium phosphate pH 8.0, 0.5 M NaCl, 50 mM imidazole). Finally, bound protein was eluted with elution buffer (50 mM sodium phosphate pH 8.0, 0.5 M NaCl, 150 mM imidazole) and colleted into two 40-ml fractions. Eluted fractions were concentrated using a Vivaspin 500 concentrator (Sartorius) with cut-off pore size of 30 kDa, and then desalted in a HiPrep 26/10 desalting column (GE Healthcare) equilibrated with 50 mM Tris-HCl, pH 8, 300 mM NaCl. Purified NifH was frozen as droplets in liquid nitrogen.

### Example 4

### Anaerobic conditions determination

Anaerobic conditions were determined by aconitase activity in cell-free extracts according to Kennedy et al. (Kennedy et al., 1983, J, Biol. Chem, 258, 11098-11105). Protein concentration was determined by bicinchoninic acid method (Pierce), with bovine serum albumin as the standard. Sodium dodecyl sulfate-polyacrylamide gel electrophoresis (SDS-PAGE) was performed as described in Laemmli (Laemmli, 1970 Nature 227:680-685). Immunoblot analysis was carried out with antibodies raised against the *A. vinelandii* NifH protein.

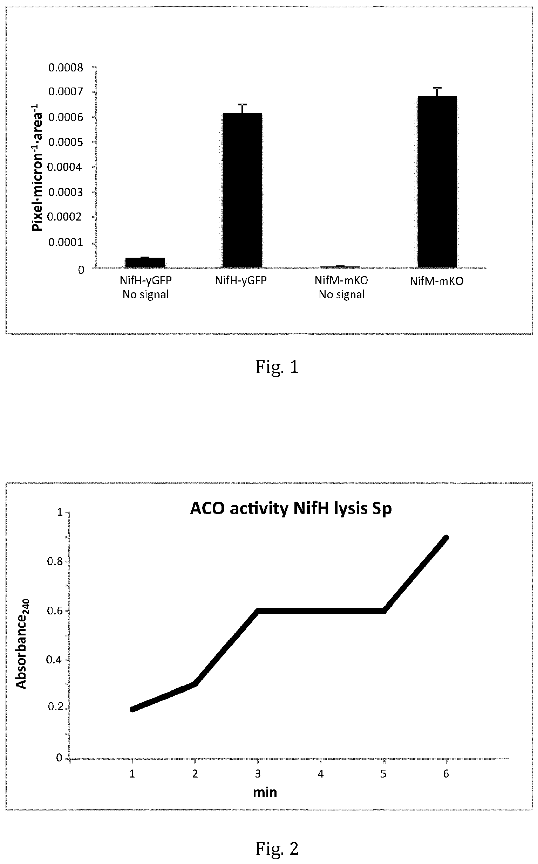

Figure 2 shows acconitase activity assay in cell-free extracts of induced GF2 cells. The presence of activity from the mitochondrial aconitase enzyme indicates the lack of oxygen stress conditions during galactose induction.

### Example 5

### Co-localization of NifH and NifM proteins in mitochondria by confocal microscopy

*S. cerevisiae* GF3 and GF4 strains were grown at 30°C in 1-liter flasks containing 500 ml of SD medium supplemented with auxotrophic requirements to an OD₆₀₀ of 0.6. Cells were then collected by centrifugation, washed with SD medium lacking glucose, resuspended in SD medium supplemented with 2% galactose to induce NifH and NifM expression, and incubated under the same conditions for 48 h.

Induced cells were collected at 4°C by centrifugation at 5,000 rpm for 5 min and then resuspended in yeast suspension buffer containing: 50 mM Tris-HCl, pH 7.5, 5 mM EDTA, 10% glycerol, and 1 mM PMSF. Whole-cell fluorescence was analyzed by using a confocal microscope Leica TCS SP8 equipped with a PL APO 40x/1,1 water immersion objective. Excitation beam splitters DD 488/514 and DD 488/561 were used to capture detailed localization images of yEGFP-NifH and NifM-mKO fusion proteins, respectively. In parallel, galactose induced cells were treated with MitoTracker Deep Red according to the manufacturer's instructions (Invitrogen) to stain mitochondria. Quantification of yEGFP and mKO fluorescence in mitochodria of induced cells is presented in Figure 1.

### Example 6

### NifH activity determination

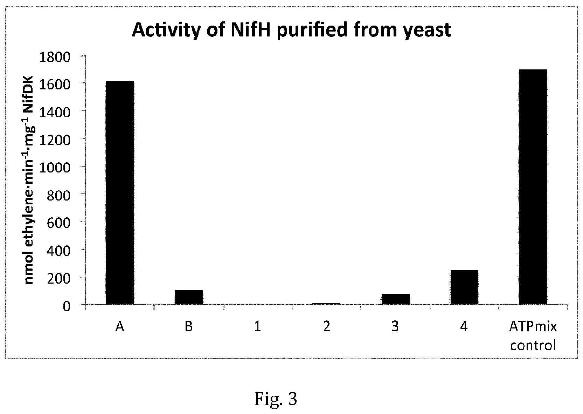

Activity of NifH preparations purified from *S. cerevisiae* GF2 cells was routinely analyzed by the acetylene reduction assay after addition of excess NifDK and ATP-regenerating mixture (1.23 mM ATP, 18 mM phosphocreatine, 2.2 mM MgCl2, 3 mM Na2S2O4, and 40 µg of creatine phosphokinase). Positive control reactions were carried out with NifH and NifDK proteins purified from *A. vinelandii* under anaerobic conditions as described in Curatti et al. (Curatti et al., 2007, Proc.Natl. Acad.Sci. USA, 104: 17626-17631). Maximum specific activity of 1600 nanomoles of ethylene formed·min⁻¹·milligram of protein⁻¹ was obtained at a NifH/NifDK molar ratio of 40 (Figure 3).

In order to quantify the NH₃ producing activity of nitrogenase with recombinant NifH, argon gas was evacuated from reaction vials and replaced by N₂ at 1 atm. Reactions were carried out at 30°C for 60 min and then stopped by addition of 50 mM EDTA. The NH₃ produced was determined by liquid chromatographic fluorescence using an Agilent 1200 HPLC and a Jasco FP-920 fluorescence detector according to Corbin et al. (Corbin et al., 1984, Appl. Envirom. Microbiol, 1027-1030). The specific activity of 869 nanomoles of ammonia formed·min⁻¹·milligram of protein⁻¹ was obtained at an apparent NifH/NifDK (from *S. cerevisiae* and *A. vinelandii,* respectively) molar ratio of 40. Positive control reactions were carried out with NifH and NifDK proteins purified from *A. vinelandii* under anaerobic conditions (See Table 1).

**Table 1: Specific activity or ammonia formed by recombinant NifH from Saccaromyces cerevisiae (_{y}NifH) and NifDK from Azotobacter vinelandii.(_{Avi}NifH/NifDK)**

| | RFU¹ | Ammonia (nmol/ml) | Specific activity (nmol of ammonia·min⁻¹·milligram of NifDK⁻¹ |
|---|---|---|---|
| _{Avi}NifH/NifDK | 1304 | 179 | 766 |
| _{y}NifH | 1456 | 200 | 869 |
| NifDK | 11 | 1,5 | 0 |
| Standard 0 | 12 | 0 | - |
| Standard 50 | 328 | 50 | - |
| Standard 100 | 731 | 100 | - |
| Standard 500 | 3623 | 500 | - |

| | | | |
|---|---|---|---|
| Standards and appropriate controls were run each time. The positive control is the maximum NifDK activity obtained at the NifH/NifDK molar ratio of 40. Both components were purified from *Azotobacter vienlandii*. Correction for the reagent background has already been made. ¹RFU: Relative Fluorescence Units. | | | |

## Claims

1. A polynucleotide encoding a fusion protein which comprises an oxygen-sensitive protein and a mitochondrial targeting peptide, wherein said oxygen-sensitive protein is NifH.

2. The polynucleotide according to claim 1, wherein said oxygen-sensitive protein is from a prokaryotic organism, more preferably said prokaryotic organism is *Azotobacter vinelandii*.

3. The polynucleotide according to claims 1 or 2, wherein said mitochondrial targeting peptide is N-terminal to the oxygen-sensitive protein, and/or preferably said mitochondrial targeting peptide comprises the mitochondrial targeting peptide of superoxide dismutase.

4. The polynucleotide according to any of claims 1 to 3, wherein the fusion protein further comprises at least one peptide tag adequate for detection or purification of the oxygen-sensitive protein.

5. The polynucleotide according to any of claims 1 to 4, wherein the sequences encoding the fusion protein have been codon optimized for expression in a eukaryotic cell, preferably in a *Saccharomyces cerevisae* cell.

6. The polynucleotide according to any of claims 1 to 5, further comprising transcriptional regulatory elements operatively linked to said polynucleotide or translational regulatory elements operatively linked to the fusion protein encoded by said polynucleotide.

7. The polynucleotide according to any of claims 1 to 6, wherein the polynucleotide further comprises a nucleotide sequence which encodes a fusion protein comprising the NifM protein and a second mitochondrial targeting peptide, preferably said second mitochondrial targeting peptide is N-terminal to NifM, and more preferably said second mitochondrial targeting peptide comprises the mitochondrial targeting peptide of superoxide dismutase.

8. The polynucleotide according to claim 7 wherein the sequences encoding the fusion protein comprising the NifM protein have been codon optimized for expression in a eukaryotic cell, preferably in a *Saccharomyces cerevisiae* cell.

9. The polynucleotide according to any of claims 7 or 8 further comprising transcriptional regulatory elements operatively linked to the polynucleotide encoding the fusion protein comprising the NifM protein and a mitochondrial targeting peptide or translational regulatory elements operatively linked to said fusion protein.

10. An expression vector comprising the polynucleotide according to any of claims 1 to 9.

11. A eukaryotic cell comprising the polynucleotide according to any of claims 1 to 9 or an expression vector according to claim 10, preferably said eukaryotic cell grows under aerobic conditions and/or more preferably said eukaryotic cell is a *Saccharomyces cerevisiae* cell.

12. Method for expressing NifH in a eukaryotic cell comprising the steps of:
i) introducing into said cell a polynucleotide according to any of claims 1 to 9 or a vector according to claim 10,
ii) growing said cell under conditions allowing the expression of said oxygen-sensitive protein and, if desired,
iii) purifying said oxygen-sensitive protein under anaerobic conditions.

13. Method for *in vitro* reconstitution of active nitrogenase protein complex comprising the steps of:
i) introducing into said cell a polynucleotide according to any of claims 1 to 9 or a vector according to claim 10, wherein the cell further comprises a nucleotide sequence which encodes a fusion protein comprising the NifM protein and a mitochondrial targeting peptide wherein said second mitochondrial targeting peptide is N-terminal to NifM,
ii) growing said cell under conditions allowing the expression of NifH and NifM proteins,
iii) purifying NifH protein from said cells under anaerobic conditions and
iv) contacting said purified NifH of step (iii) with the NifDK complex;
preferably the nucleotide sequence which encodes a fusion protein comprising the NifM protein and a mitochondrial targeting peptide is provided in the polynucleotide that comprises the nucleotide sequence which encodes a fusion protein comprising the NifH protein and a mitochondrial targeting peptide; more preferably said NifDK complex is isolated from *Azotobacter vinelandii*. and/or still more preferably the NifH/NifDK molar ratio is of at least 10.

14. Use of a polynucleotide according to any of claims 1 to 9, an expression vector according to claim 10 or a eukaryotic cell according to claim 11 for expressing NifH.

15. A kit comprising:
i) a polynucleotide according to any of claims 1 to 9 or an expression vector according to claim 10;
ii) reagents adequate for carrying a method according to any of claims 12 or 13; and
iii) preferably further comprising a nitrogenase substrate.

## Patentansprüche

1. Polynukleotid, welches ein Fusionsprotein kodiert, das ein sauerstoffempfindliches Protein sowie ein mitochondrial zielfsteuerndes Peptid umfasst, wobei das sauerstoffempfindliche Protein NifH ist.

2. Polynukleotid nach Anspruch 1, wobei das sauerstoffempfindliche Protein aus einem prokaryontischen Organismus stammt, wobei der prokaryontische Organismus besonders bevorzugt *Azotobacter vinelandii* ist.

3. Polynukleotid nach Anspruch 1 oder 2, wobei das mitochondrial zielsteuernde Peptid N-Terminal zu dem sauerstoffempfindlichen Proteins ist, und/oder vorzugsweise das mitochondrial zielfsteuernde Peptid das mitochondrial zielfsteuernde Peptid von Superoxid-Dismutase enthält.

4. Polynukleotid nach einem der Ansprüche 1 bis 3, wobei das Fusionsprotein desweitern wenigstens eine Peptidmarkierung enthält, welche zur Detektion oder Reinigung des sauerstoffempfindlichen Proteins geeignet ist.

5. Polynukleotid nach einem der Ansprüche 1 bis 4, wobei die das Fusionsprotein kodierenden Sequenzen zur Expression in einer eukaryontischen Zelle, vorzugsweise in einer *Saccharomyces cerevisiae* - Zelle, codon-optimiert worden sind.

6. Polynukleotid nach einem der Ansprüche 1 bis 5, welches desweitern transkriptionell regulatorische Elemente, die mit dem Polynukleotid funktionell verbunden sind, oder translational regulatorische Elemente umfasst, die mit dem durch das Polynukleotid kodierte Fusionsprotein funktionell verbunden sind.

7. Polynukleotid nach einem der Ansprüche 1 bis 6, wobei das Polynukleotid desweitern eine Nukleotidsequenz umfasst, welche ein Fusionsprotein kodiert, welches das NifM-Protein und ein zweites mitochondriales zielfsteuerndes Peptid umfasst, wobei vorzugsweise das zweite mitochondrial zielfsteuernde Peptid N-terminal zu dem NifM ist und besonders bevorzugt das zweite mitochondrial zielfsteuernde Peptid das mitochondrial zielfsteuernde Peptid von Superoxid-Dismutase enthält.

8. Polynukleotid nach Anspruch 7, wobei die das das NifM-Protein umfassende Fusionsprotein kodierenden Sequenzen zur Expression in einer eukaryontischen Zelle, vorzugsweise in einer *Saccharomyces cerevisiae*-Zelle codon-optimiert worden ist.

9. Polynukleotid nach einem der Ansprüche 7 oder 8, welches desweitern transkriptionell regulatorische Elemente, die mit dem das das NifM-Protein umfassenden Fusionsprotein kodierenden Polynukleotid funktionell verbunden sind, sowie ein mitochondrial zielfsteuerndes Peptid oder translationell regulatorische Elemente umfasst, die mit dem Fusionsproteinen funktionell verbunden sind.

10. Expressionsvektor umfassend ein Polynukleotid nach einem der Ansprüche 1 bis 9.

11. Eukaryontische Zelle umfassend ein Polynukleotid nach einem der Ansprüche 1 bis 9 oder einen Expressionsvektor nach Anspruch 10, wobei vorzugsweise die eukaryontische Zelle unter aeroben Bedingungen wächst und/oder besonders bevorzugt die eukaryontische Zelle eine *Saccharomyces cerevisiae*-Zelle ist.

12. Verfahren zum Exprimieren von NifH in einer eukaryontischen Zelle umfassend die Schritte:
i) Einführen eines Polynukleotids nach einem der Ansprüche 1 bis 9 oder eines Vektors nach Anspruch 10 in die Zelle,
ii) Wachsen der Zelle unter Bedingungen, welche die Expression des sauerstoffempfindlichen Proteins erlauben, und gegebenenfalls
iii) Reinigen des sauerstoffempfindlichen Proteins unter anaeroben Bedingungen.

13. Verfahren zur *in vitro*-Rekonstitution von aktivem Nitrogenase-Protein-Komplex umfassend die Schritte:
i) Einführen eines Polynukleotids nach einem der Ansprüche 1 bis 9 oder eines Vektors nach Anspruch 10 in die Zelle, wobei die Zelle desweitern eine Nukleotidsequenz umfasst, welche ein das NifM-Protein umfassendes Fusionsprotein und ein mitochondrial zielfsteuerndes Peptid kodiert, wobei das zweite mitochondrial zielsteuernde Peptid N-terminal zu NifM ist,
ii) Wachsen der Zelle unter Bedingungen, welche die Expression von NifH- und NifM-Proteinen erlaubt,
iii) Reinigen von NifH-Protein von den Zellen unter anaeroben Bedingungen und
iv) Kontaktieren des gereinigten NifH von Schritt iii) mit dem NifDK-Komplex,
wobei vorzugsweise die Nukleotidsequenz, welche ein Fusionsprotein kodiert, welches das NifM-Protein und ein mitochondrial zielsteuerndes Peptid umfasst, in dem Polynukleotid vorgesehen wird, das die Nukleotidsequenz umfasst, welches ein Fusionsprotein kodiert, welches das NifH-Protein und ein mitochondrial zielsteuerndes Peptid umfasst, wobei besonders bevorzugt der NifDK-Komplex aus *Azotobacter vinelandii* isoliert wird, und/oder, wobei besonders bevorzugt das molare Verhältnis von NifH/NifDK wenigstens 10 beträgt.

14. Verwendung eines Polynukleotids nach einem der Ansprüche 1 bis 9, eines Expressionsvektors nach Anspruch 10 oder einer eukaryontischen Zelle nach Anspruch 11 zum Exprimieren von NifH.

15. Kit umfassend:
i) ein Polynukleotid nach einem der Ansprüche 1 bis 9 oder einen Expressionsvektor nach Anspruch 10,
ii) Reagenzien, welche zum Durchführen eines Verfahrens nach einem der Ansprüche 12 oder 13 geeignet sind, und
iii) vorzugsweise desweitern umfassend ein Nitrogenase-Substrat.

## Revendications

1. Un polynucléotide codant pour une protéine de fusion qui comprend une protéine sensible à l'oxygène et un peptide de ciblage mitochondrial, ladite protéine sensible à l'oxygène étant une NifH.

2. Le polynucléotide selon la revendication 1, dans lequel ladite protéine sensible à l'oxygène provient d'un organisme procaryote, plus préférablement ledit organisme procaryote est *Azotobacter vinelandii.*

3. Le polynucléotide selon la revendication 1 ou la revendication 2, dans lequel ledit peptide de ciblage mitochondrial est N-terminal à la protéine sensible à l'oxygène, et / ou de préférence ledit peptide de ciblage mitochondrial comprend le peptide de ciblage mitochondrial de la superoxyde dismutase.

4. Le polynucléotide selon l'une quelconque des revendications 1 à 3, dans lequel la protéine de fusion comprend en outre au moins une étiquette peptidique adéquate pour la détection ou la purification de la protéine sensible à l'oxygène.

5. Le polynucléotide selon l'une quelconque des revendications 1 à 4, dans lequel les séquences codant pour la protéine de fusion ont été optimisées pour l'expression dans une cellule eucaryote, de préférence dans une cellule de *Saccharomyces cerevisae*.

6. Le polynucléotide selon l'une quelconque des revendications 1 à 5, comprenant en outre des éléments de régulation de la transcription liés de manière fonctionnelle audit polynucléotide ou des éléments régulateurs de la transcription liés de manière fonctionnelle à la protéine de fusion codée par ledit polynucléotide.

7. Le polynucléotide selon l'une quelconque des revendications 1 à 6, dans lequel le polynucléotide comprend en outre une séquence nucléotidique qui code une protéine de fusion comprenant la protéine NifM et un deuxième peptide de ciblage mitochondrial, ledit deuxième peptide de ciblage mitochondrial est de préférence N-terminal à NifM, et plus préférablement ledit deuxième peptide de ciblage mitochondrial comprend le peptide de ciblage mitochondrial de la superoxyde dismutase.

8. Le polynucléotide selon la revendication 7, dans lequel les séquences codant pour la protéine de fusion comprenant la protéine NifM ont été optimisées pour l'expression dans une cellule eucaryote, de préférence dans une cellule de *Saccharomyces cerevisiae*.

9. Le polynucléotide selon l'une quelconque des revendications 7 ou 8, comprenant en outre des éléments de régulation de la transcription liés de manière fonctionnelle au polynucléotide codant la protéine de fusion comprenant la protéine NifM et un peptide de ciblage mitochondrial ou des éléments régulateurs de transcription liés de manière fonctionnelle à ladite protéine de fusion.

10. Un vecteur d'expression comprenant le polynucléotide selon l'une quelconque des revendications 1 à 9.

11. Une cellule eucaryote comprenant le polynucléotide selon l'une quelconque des revendications 1 à 9 ou un vecteur d'expression selon la revendication 10, de préférence ladite cellule eucaryote se développe dans des conditions aérobies et / ou plus préférablement ladite cellule eucaryote est une cellule de *Saccharomyces cerevisiae*.

12. Procédé pour exprimer NifH dans une cellule eucaryote, comprenant les étapes consistant à :
i) introduire dans ladite cellule un polynucléotide selon l'une quelconque des revendications 1 à 9 ou un vecteur selon la revendication 10,
ii) faire croître ladite cellule dans des conditions permettant l'expression de ladite protéine sensible à l'oxygène et, si désiré,
iii) purifier ladite protéine sensible à l'oxygène dans des conditions anaérobies.

13. Procédé de reconstitution *in vitro* d'un complexe protéique nitrogénase actif comprenant les étapes consistant à :
i) introduire dans ladite cellule un polynucléotide selon l'une quelconque des revendications 1 à 9 ou un vecteur selon la revendication 10, la cellule comprenant en outre une séquence nucléotidique qui code une protéine de fusion comprenant la protéine NifM et un peptide de ciblage mitochondrial, ledit deuxième peptide de ciblage mitochondrial étant N-terminal à NifM,
ii) faire croître ladite cellule dans des conditions permettant l'expression des protéines NifH et NifM,
iii) purifier la protéine NifH desdites cellules dans des conditions anaérobies, et
iv) mettre en contact ledit NifH purifié de l'étape (iii) avec le complexe NifDK ;
de préférence la séquence nucléotidique qui code une protéine de fusion comprenant la protéine NifM et un peptide de ciblage mitochondrial est fournie dans le polynucléotide qui comprend la séquence nucléotidique qui code une protéine de fusion comprenant la protéine NifH et un peptide de ciblage mitochondrial ; plus préférablement, ledit complexe NifDK est isolé à partir d'*Azotobacter vinelandii*. et / ou encore plus préférentiellement, le rapport molaire NifH / NifDK est d'au moins 10.

14. Utilisation d'un polynucléotide selon l'une quelconque des revendications 1 à 9, d'un vecteur d'expression selon la revendication 10 ou d'une cellule eucaryote selon la revendication 11 pour l'expression de NifH.

15. Un kit comprenant :
i) un polynucléotide selon l'une quelconque des revendications 1 à 9 ou un vecteur d'expression selon la revendication 10 ;
ii) des réactifs adéquats pour mettre en oeuvre un procédé selon l'une quelconque des revendications 12 ou 13 ; et
iii) de préférence comprenant de plus un substrat de nitrogénase.
